(19) Europäisches Patentamt
European Patent Office
Office européen des brevets

(11) **EP 4 614 516 A1**

(12) **EUROPEAN PATENT APPLICATION**

(43) Date of publication:
10.09.2025 Bulletin 2025/37

(21) Application number: 25161457.4

(22) Date of filing: 04.03.2025

(51) International Patent Classification (IPC):
**G16H 50/20** (2018.01)   **A61B 5/0533** (2021.01)
**A61B 5/16** (2006.01)   G06N 3/08 (2023.01)

(52) Cooperative Patent Classification (CPC):
**G16H 50/20; A61B 5/0533; A61B 5/165;**
**A61B 5/7267;** A61B 5/164; G06N 3/08;
G16H 20/30; G16H 20/70; G16H 50/30

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 06.03.2024 IN 202421015894

(71) Applicant: Tata Consultancy Services Limited
Maharashtra (IN)

(72) Inventors:
• MUKHOPADHYAY, Shalini
700135 Kolkata, West Bengal (IN)
• JAISWAL, Dibyanshu
700135 Kolkata, West Bengal (IN)
• SHARMA, Varsha
453112 Indore, Madhya Pradesh (IN)
• DEY, Swarnava
700135 Kolkata, West Bengal (IN)
• GHOSE, Avik
700135 Kolkata, West Bengal (IN)

(74) Representative: Goddar, Heinz J.
Boehmert & Boehmert
Anwaltspartnerschaft mbB
Pettenkoferstrasse 22
80336 München (DE)

(54) **FEATURE OPTIMIZATION AND AUTOMATED GENERATION OF TINY MODELS FOR AFFECTIVE PARAMETER DETERMINATION FROM ELECTRODERMAL ACTIVITY**

(57) Wearable sensor-based stress detection is a well explored area of research in affective computing domain and are performed with non-invasive sensing modalities like Electrodermal Activity (EDA). In recent years, with the increased availability of such wearable devices to end-users, these applications have become more pervasive and thus require a greater level of optimization for continuous usage on resource constrained and battery-powered devices. While several research works have focused on designing Machine Learning (ML) and Deep Learning (DL) models for these tasks, very few focus on resource footprint. The balance between classification accuracy and computational resources is difficult to achieve with manual design process and is time consuming. In the present disclosure, systems and methods apply Neural Network Search Space (NASS) technique on features for feature optimization, and generation of tiny models based on optimized features set suitable for round-the-clock inference, with minimal resource requirements, and low latency.

FIG. 3

**Description**

CROSS-REFERENCE TO RELATED APPLICATIONS AND PRIORITY

The present application claims priority to Indian application no. 202421015894, filed on March 6, 2024.

TECHNICAL FIELD

The disclosure herein generally relates to feature optimization and tiny models, and, more particularly, to feature optimization and automated generation of tiny models for affective parameter determination from electrodermal activity.

BACKGROUND

Wearable sensor-based stress detection is a well explored area of research in the domain of affective computing and can be performed with the help of non-invasive sensing modalities like Electrodermal Activity (EDA). The EDA sensors with a wearable form factor are commonly available on commercial off-the-shelf devices. In recent years, with the increased availability of such wearable devices to end-users, these applications have become more pervasive and thus require a greater level of optimization for continuous usage on resource constrained and battery-powered devices. While several research works have focused on designing Machine Learning (ML) and Deep Learning (DL) models for these tasks, very few focus on the resource footprint of these models. The balance between classification accuracy and computational resources is difficult to achieve with a manual design process and takes a long time.

SUMMARY

Embodiments of the present disclosure present technological improvements as solutions to one or more of the above-mentioned technical problems recognized by the inventors in conventional systems.
For example, in one aspect, there is provided a processor implemented method for feature optimization and automated generation of tiny models for affective parameter determination from electrodermal activity. The method comprises receiving, via one or more hardware processors, a raw Electrodermal Activity (EDA) signal from a wearable device, wherein the raw Electrodermal Activity (EDA) signal pertains to a user; normalizing, via the one or more hardware processors, the raw EDA signal to obtain a normalized EDA signal; extracting, via the one or more hardware processors, a set of features from the normalized EDA signal; estimating, via the one or more hardware processors, a computation overhead for each feature amongst the set of features; processing, by using a Neural Network Search Space (NNSS) via the one or more hardware processors, the raw EDA signal, the normalized EDA signal, the set of features, and the computation overhead to obtain an optimized set of features, wherein a feature objective function is configured to process the computation overhead of each feature to obtain the optimized set of features; generating, by using the Neural Network Search Space (NNSS) via the one or more hardware processors, one or more classification models using the optimized set of features; and selecting, via the one or more hardware processors, at least one classification model amongst the one or more classification models based on a metric objective function obtained from the associated performance metrics.
In an embodiment, the step of normalizing the raw EDA signal comprises determining one or more baseline components in the raw EDA signal; and computing mean and standard deviation for the one or more baseline components based on presence of an activity or stimuli in the raw EDA signal to obtain the normalized EDA signal.
In an embodiment, the method further comprises determining in real-time at the wearable device, by using the at least one selected classification model deployed therein, a presence of a graded level of an affective parameter in the user based on an EDA signal being captured.
In an embodiment, the affective parameter comprises at least one of stress, anxiety, emotion, cognitive load, attention, and valence.
In an embodiment, the set of features comprises at least one of one or more Tonic features, one or more Phasic features, a ratio of a mean associated with the one or more Tonic features and the one or more Phasic features, an entropy ratio of the one or more Tonic and the one or more Phasic features, a Phasic features peak from an associated baseline and an associated threshold, a Tonic features peak from an associated baseline and an associated threshold, a fractal dimension of the one or more Phasic features, a skin conductance stimuli, an associated slope obtained from the normalized EDA signal, and one or more frequency domain features.
In another aspect, there is provided a processor implemented system for feature optimization and automated generation of tiny models for affective parameter determination from electrodermal activity. The system comprises a memory storing instructions; one or more communication interfaces; and one or more hardware processors coupled to the memory via the one or more communication interfaces, wherein the one or more hardware processors are configured by the instructions to receive a raw Electrodermal Activity (EDA) signal from a wearable device, wherein the raw Electrodermal Activity (EDA) signal pertains to a user; normalize the raw EDA signal to obtain a normalized EDA signal; extract a set of features from the normalized EDA signal; estimate a computation overhead for each feature amongst the set of features; process, by using a Neural Network Search Space (NNSS), the raw EDA signal, the normalized EDA signal, the set of features, and the computation overhead to obtain an optimized set of features, wherein a feature objective function is configured to process the computation overhead of each feature to obtain the optimized set of features; generate, by using the Neural Network Search Space (NNSS), one or more classification models using the optimized set of features; and select at least one classification model amongst the one or more classification models based on a metric objective function obtained from the associated performance metrics.

In an embodiment, the raw EDA signal is normalized by determining one or more baseline components in the raw EDA signal; and computing mean and standard deviation for the one or more baseline components based on presence of an activity or stimuli in the raw EDA signal to obtain the normalized EDA signal.

In an embodiment, the one or more hardware processors are further configured by the instructions to determine in real-time at the wearable device, by using the at least one selected classification model deployed therein, a presence of a graded level of an affective parameter in the user based on an EDA signal being captured.

In an embodiment, the affective parameter comprises at least one of stress, anxiety, emotion, cognitive load, attention, and valence.

In an embodiment, the set of features comprises at least one of one or more Tonic features, one or more Phasic features, a ratio of a mean associated with the one or more Tonic features and the one or more Phasic features, an entropy ratio of the one or more Tonic and the one or more Phasic features, a Phasic features peak from an associated baseline and an associated threshold, a Tonic features peak from an associated baseline and an associated threshold, a fractal dimension of the one or more Phasic features, a skin conductance stimuli, an associated slope obtained from the normalized EDA signal, and one or more frequency domain features.

In yet another aspect, there are provided one or more non-transitory machine-readable information storage mediums comprising one or more instructions which when executed by one or more hardware processors cause feature optimization and automated generation of tiny models for affective parameter determination from electrodermal activity by receiving a raw Electrodermal Activity (EDA) signal from a wearable device, wherein the raw Electrodermal Activity (EDA) signal pertains to a user; normalizing the raw EDA signal to obtain a normalized EDA signal; extracting a set of features from the normalized EDA signal; estimating a computation overhead for each feature amongst the set of features; processing, by using a Neural Network Search Space (NNSS), the raw EDA signal, the normalized EDA signal, the set of features, and the computation overhead to obtain an optimized set of features, wherein a feature objective function is configured to process the computation overhead of each feature to obtain the optimized set of features; generating, by using the Neural Network Search Space (NNSS), one or more classification models using the optimized set of features; and selecting, at least one classification model amongst the one or more classification models based on a metric objective function obtained from the associated performance metrics.

In an embodiment, the step of normalizing the raw EDA signal comprises determining one or more baseline components in the raw EDA signal; and computing mean and standard deviation for the one or more baseline components based on presence of an activity or stimuli in the raw EDA signal to obtain the normalized EDA signal.

In an embodiment, the one or more instructions which when executed by the one or more hardware processors further cause determining in real-time at the wearable device, by using the at least one selected classification model deployed therein, a presence of a graded level of an affective parameter in the user based on an EDA signal being captured.

In an embodiment, the affective parameter comprises at least one of stress, anxiety, emotion, cognitive load, attention, and valence.

In an embodiment, the set of features comprises at least one of one or more Tonic features, one or more Phasic features, a ratio of a mean associated with the one or more Tonic features and the one or more Phasic features, an entropy ratio of the one or more Tonic and the one or more Phasic features, a Phasic features peak from an associated baseline and an associated threshold, a Tonic features peak from an associated baseline and an associated threshold, a fractal dimension of the one or more Phasic features, a skin conductance stimuli, an associated slope obtained from the normalized EDA signal, and one or more frequency domain features.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 depicts an exemplary system for feature optimization and automated generation of tiny models for affective parameter determination from electrodermal activity (EDA), in accordance with an embodiment of the present disclosure.

FIG. 2 depicts an exemplary high level block diagram of the system of FIG. 1 for feature optimization and automated generation of tiny models for affective parameter determination from electrodermal activity, in accordance with an embodiment of the present disclosure.

FIG. 3 depicts an exemplary high level block diagram of the system of FIG. 1 for feature optimization and automated generation of tiny models for affective parameter determination from electrodermal activity, in accordance with an embodiment of the present disclosure.

FIG. 4 depicts an exemplary flow chart illustrating a method for feature optimization and automated generation of tiny models for affective parameter determination from electrodermal activity, using the systems of FIGS. 1-2, in accordance with an embodiment of the present disclosure.

FIGS. 5A through 5D depict a graphical representation illustrating performance metrics for stress detection model generation (e.g., classification model) with a Neural Network Search Space (NNSS) (or Neural Architecture Search - NAS) in accordance with an embodiment of the present disclosure.

FIG. 6 depicts auto-generated tiny models (e.g., classification models) by the system of FIGS. 1-2, in accordance with an embodiment of the present disclosure.

FIGS. 7A through 7C depict a graphical representation illustrating selected time series features from a feature space (as opposed to features with one single value for a window), in accordance with an embodiment of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Stress is a physiological and psychological response to challenges or perceived threats, which can manifest as acute (short-term) or chronic (long-term) effects (e.g., refer "H. Selye, Stress in health and disease. Butterworth-Heinemann, 2013."). It involves the body's reaction to pressure, stemming from external or internal sources such as work, relationships, or life events. In Gallup's State of the Global Workplace 2023 Report (e.g., refer "Gallup, "State-of-the-global-workplace," https://www.gallup.com/workplace/349484/state-of-the-globalworkplace.aspx#ite-506924, 2023, [Online; accessed 30-November-2023]."), East Asia had the same stress level as the U.S. and Canada, reaching 52%. Australia and New Zealand followed closely at 47%. This data underscores how stress has become an enduring aspect of contemporary living. Within affective computing, electrodermal activity (EDA), a physiological measure of sympathetic nervous system activity, has demonstrated potential for non-invasive and continuous stress monitoring (e.g., refer "W. Boucsein, Electrodermal activity. Springer Science & Business Media, 2012."). EDA is also referred to as galvanic skin response (GSR) and it measures the variations in electrical conductance of the skin as a response to sweat production. It is measured by applying a low level of constant voltage to the skin surface followed by measurement of the change in skin conductance. EDA is linked with the regulation of internal temperatures, as well as with emotional arousal. The EDA signal has been observed to reflect the intensity of a user's emotional state, also known as emotional arousal. Real-time stress prediction holds immense value across various domains, contributing to overall well-being. Firstly, it enables individuals to become more self-aware of their stress levels, empowering them to take proactive measures in managing stress before it progresses into severe conditions like anxiety or depression. Additionally, it facilitates the continuous monitoring of patients' stress levels, particularly benefiting those with chronic conditions like heart disease or hypertension, conditions exacerbated by stress.

Understanding stress through wearable devices is pivotal for developing better tools and aiding individuals. However, further exploration is required to develop more advanced and precise methods for prompt stress prediction, especially considering individual differences. Additionally, the scarcity of studies tracking stress and physiological signals in the long term poses a challenge in assessing the sustained effectiveness of stress prediction models. Ethical considerations, including privacy concerns and user perceptions regarding the use of these devices for stress monitoring, demand careful attention. Therefore, to ensure confidentiality, data should be analyzed on the device itself rather than on cloud-based or other server-based systems. Keeping this in mind, the system of the present disclosure is depicted herein where various sources of stress in daily life, and the framework to address this problem efficiently are described.

The approach of the present disclosure is to employ an automation framework, a Neural Architecture Search (NAS) technique (or also referred to as Neural Network Search Space (NNSS) technique and interchangeably used herein), for the quick generation of classification models (e.g., say stress detection models) customized for any specified wearable device. With the easy availability of a wide range of devices having diverse compute capacities and battery limitations, it is necessary to devise a low-footprint system to help expedite the generation of these classification networks whenever the user's device changes. This allows for the best utilization of available resources on the device while assuring optimized classification accuracy and battery power consumption. The disclosed system creates tiny stress classification models in the kilobyte range (< 100 kB) while providing accuracy comparable to state-of-the-art (SoA) techniques.

Furthermore, since the specified lightweight neural network search space (NNSS) algorithm/technique runs on any standard CPU-based system, it eliminates the requirement of powerful, GPU-based computing systems to generate wearable sensor-based tiny classification networks. Existing approaches for Affective Computing, Stress detection, and Auto-generation of Neural Network Models. Furthermore, there are limited prior works discussing NNSS techniques that are capable of generating optimized models quickly, without the requirement of GPU-enabled computing systems.

Affective Computing focuses on understanding and responding to human emotions. Picard (e.g., refer "R. W. Picard, Affective computing. MIT press, 2000.") laid the groundwork, emphasizing the importance of emotional intelligence in human-computer interaction. Some advancements include the development of multimodal emotion recognition systems (e.g., refer "M. Pantic and L. J. Rothkrantz, "Toward an affect-sensitive multimodal human-computer interaction," Proceedings of the IEEE, vol. 91, no. 9, pp. 1370-1390, 2003.") and affect-aware intelligent systems (e.g., refer "R. A. Calvo and S. D'Mello, "Frontiers of affect-aware learning technologies," IEEE Intelligent Systems, vol. 27, no. 6, pp. 86-89, 2012.", and "P. Schmidt, A. Reiss, R. D··urichen, and K. Van Laerhoven, "Wearable-based affect recognition-a review," Sensors, vol. 19, no. 19, p. 4079, 2019."). In the broader spectrum, stress detection is crucial in assessing individuals' emotional states and well-being. Traditional approaches often relied on physiological measures like heart rate variability and self-reported instances. However, these methods pose limitations in terms of accuracy and real-time monitoring capabilities. The integration of computational techniques, especially deep learning, has shown promise in advancing stress detection methodologies due to their ability to process complex data and extract intricate patterns. Recent studies by R Li et al (e.g., refer "R. Li and Z. Liu, "Stress detection using deep neural networks," BMC Medical Informatics and Decision Making, vol. 20, pp. 1-10, 2020.") and P Bobade et al (e.g., refer "P. Bobade and M. Vani, "Stress detection with machine learning and deep learning using multimodal physiological data," in 2020 Second International Conference on Inventive Research in Computing Applications (ICIRCA). IEEE, 2020, pp. 51-57.") demonstrate the efficacy of deep learning models in detecting stress from physiological signals or behavioral data. The shift towards wearable-based stress detection systems is evident. Studies (e.g., refer "C. Setz, B. Arnrich, J. Schumm, R. La Marca, G. Tr··oster, and U. Ehlert, "Discriminating stress from cognitive load using a wearable eda device," IEEE Transactions on information technology in biomedicine, vol. 14, no. 2, pp. 410-417, 2009.", "S. A. Khowaja, A. G. Prabono, F. Setiawan, B. N. Yahya, and S.-L. Lee, "Toward soft real-time stress detection using wrist-worn devices for human workspaces," Soft Computing, vol. 25, pp. 2793-2820, 2021.", and "L. Ciabattoni, F. Ferracuti, S. Longhi, L. Pepa, L. Romeo, and F. Verdini, "Real-time mental stress detection based on smartwatch," in 2017 IEEE International Conference on Consumer Electronics (ICCE). IEEE, 2017, pp. 110-111.") utilized physiological data from wearable devices. However, their detection method is conducted offline and has not been implemented on a wearable device.

Neural architecture significantly influences data features and performance. However, designing neural architecture heavily relies on researchers' knowledge, presenting challenges in breaking away from established paradigms. To address this, minimizing human intervention through revolutionary algorithms like NAS/NNSS proves effective in automating deep learning network design (e.g., refer "F. Hutter, L. Kotthoff, and J. Vanschoren, Automated machine learning: methods, systems, challenges. Springer Nature, 2019."). Another work (e.g., refer "B. Zoph and Q. V. Le, "Neural architecture search with reinforcement learning," arXiv preprint arXiv:1611.01578, 2016.") proposed NAS in image classification and object detection. MetaQNN (e.g., refer "B. Baker, O. Gupta, N. Naik, and R. Raskar, "Designing neural network architectures using reinforcement learning," 2016.") employs Q-learning within a Markov decision process for optimal neural architecture selection. Large-scale Evolution (e.g., "E. Real, S. Moore, A. Selle, S. Saxena, Y. L. Suematsu, J. Tan, Q. V. Le, and A. Kurakin, "Large-scale evolution of image classifiers," in international conference on machine learning. PMLR, 2017, pp. 2902-2911.") utilized evolutionary algorithms to automatically learn the optimal neural architecture. DNA (e.g., refer "C. Li, J. Peng, L. Yuan, G. Wang, X. Liang, L. Lin, and X. Chang, "Block-wisely supervised neural architecture search with knowledge distillation," in Proceedings of the IEEE/CVF Conference on Computer Vision and Pattern Recognition, 2020, pp. 1989-1998.") modularized the NAS search space, addressing representation shift via full candidate architecture training. GDAS-NSAS (e.g., refer "M. Zhang, H. Li, S. Pan, X. Chang, and S. Su, "Overcoming multi-model forgetting in one-shot NAS with diversity maximization," in Proceedings of the IEEE/CVF Conference on Computer Vision and Pattern Recognition, 2020, pp. 7809-7818.") introduces the Novelty Search-based Architecture Selection (NSAS) loss function, tackling multi-model forgetting during super network training caused by weight sharing. Several recent studies on Time Series Classification (TSC) employing NAS include the robust neural temporal search (RNTS) framework for TSC by Xiao et al. (e.g., refer "Z. Xiao, X. Xu, H. Xing, R. Qu, F. Song, and B. Zhao, "Rnts: robust neural temporal search for time series classification," in 2021 International Joint Conference on Neural Networks (IJCNN). IEEE, 2021, pp. 1-8."), repeated k-fold cross-validation based training of deep residual networks by Rakhshani et al. (e.g., refer "H. Rakhshani, H. I. Fawaz, L. Idoumghar, G. Forestier, J. Lepagnot, J. Weber, M. Br'evilliers, and P.-A. Muller, "Neural architecture search for time series classification," in 2020 International Joint Conference on Neural Networks (IJCNN). IEEE, 2020, pp. 1-8.") and Ren et al.'s (e.g., refer "Y. Ren, L. Li, X. Yang, and J. Zhou, "Autotransformer: Automatic transformer architecture design for time series classification," in Pacific-Asia Conference on Knowledge Discovery and Data Mining. Springer, 2022, pp. 143-155.") proposal of a data-driven automatic Transformer architecture for TSC. Moreover, Mukhopadhyay et al. (e.g., refer "S. Mukhopadhyay, S. Dey, P. Ghose, A. ans Singh, and P. Dasgupta, "Generating tiny deep neural networks for ecg classification on microcontrollers," in Proceedings of the 2023 IEEE International Conference on Pervasive Computing and Communications Workshops and other Affiliated Events (PerCom Workshops): PerCom Industry Track 2023, 2023.") and Sharma et al. (e.g., "V. Sharma, S. Mukhopadhyay, S. Bhattacharya, S. Dey, and A. Ghose, "Puffconv: A system for online and on-device puff detection for smoking cessation," in 2023 IEEE International Conference on Pervasive Computing and Communications Workshops and other Affiliated Events (PerCom Workshops). IEEE, 2023, pp. 595-600.") utilizes NAS to develop compact ECG signal classification models and smoking puff detection systems for wearable platforms respectively.

Embodiments of the present disclosure provide systems and methods for feature optimization and automated generation of tiny models for affective parameter determination from electrodermal activity (e.g., say for the classification of Stress versus No-Stress), leveraging publicly available datasets. The present disclosure aims to benchmark the findings of the system and methods described herein with reference to a state-of-the-art (SoA) machine learning-based model, which is built on the premises of 4 publicly available datasets. More specifically, the present disclosure provides:

1. A system for pervasive and on-device affective parameter determination and analysis (e.g., stress detection and analysis) to ensure minimal consumption of battery power on wearable devices and preservation of user data privacy.
2. A set of unique, customized, auto-generated light-weight models obtained via a scaled, on-the-go NAS/NNSS technique, with optimal resource footprint, and low Multiply-Accumulate operations in the range of tens of Million (< 6 M)
3. Resultant models with size ranging in kilobytes (< 100 kB), with a reduction of as much as 90% compared to traditional machine learning or deep learning-based models that achieve similar performance in classification.

The additional advantage of using the auto-generated models is the simplicity of model structure and hardware support of the models on tiny microcontroller-based platforms. These models are generated with basic Neural Network Layers, such as Convolution, Max Pooling, Dense, Batch Normalization and Dropout, and thus, can be deployed directly on limited-support MCU platforms. Based on platform limitations, the search algorithm can be configured and constrained, so that there is support for a wide range of wearable devices of varying make and specifications.

More specifically, the present disclosure provides a system to detect and classify affective parameters (e.g., stress from wearable EDA data) and perform all analysis on the premises of the user's personal device itself. The system of the present disclosure follows a Deep Neural Network (DNN) based approach to achieve the same with the best efficiency with respect to system effectiveness, as well as design time. The present disclosure utilizes DNN-based models designed by domain expert researchers, and at the same time, the system of the present disclosure explores the utility of employing automation techniques to expedite the process of optimization, for quicker deployment on wearable and edge platforms.

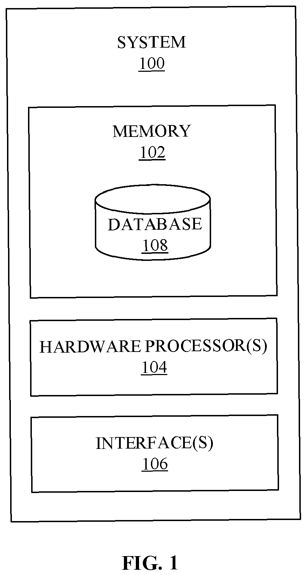

Referring now to the drawings, and more particularly to FIGS. 1 through 7C, where similar reference characters denote corresponding features consistently throughout the figures, there are shown preferred embodiments, and these embodiments are described in the context of the following exemplary system and/or method.

FIG. 1 depicts an exemplary system 100 for feature optimization and automated generation of tiny models for affective parameter determination from electrodermal activity (EDA), in accordance with an embodiment of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises information raw Electrodermal Activity (EDA) signal from a wearable device associated with a user, a normalized EDA signal, a set of features extracted, computation overhead for each feature amongst the set of features from the normalized EDA signal, and the like. The database 108 further comprises optimized set of features, one or more classification models (also referred to as tiny models (or tiny stress models) and interchangeably used herein), feature objective function, metric objective function, model's associated performance metrics (e.g., accuracy, model size, etc.), affective parameter in the user being determined in real-time, and the like. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

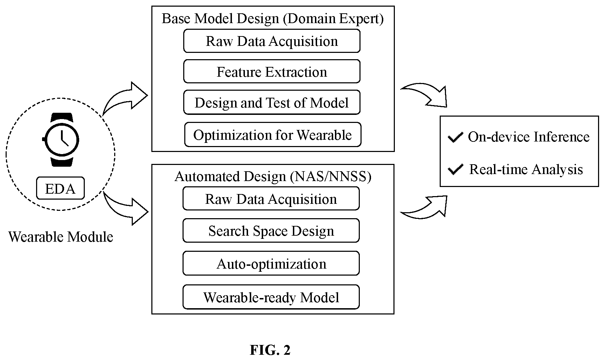

FIG. 2, with reference to FIG. 1, depicts an exemplary high level block diagram of the system 100 of FIG. 1 for feature optimization and automated generation of tiny models for affective parameter determination from electrodermal activity, in accordance with an embodiment of the present disclosure.

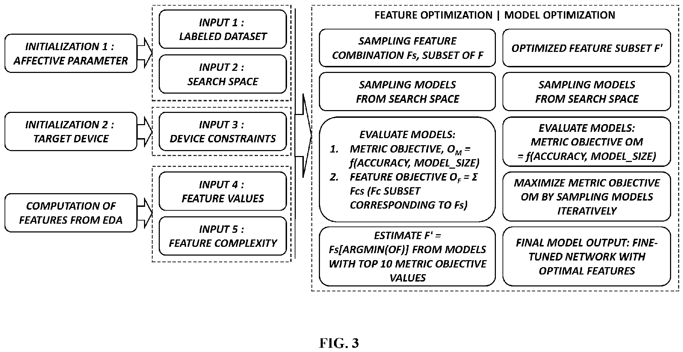

FIG. 3, with reference to FIGS. 1-2, depicts an exemplary high level block diagram of the system 100 of FIG. 1 for feature optimization and automated generation of tiny models for affective parameter determination from electrodermal activity, in accordance with an embodiment of the present disclosure.

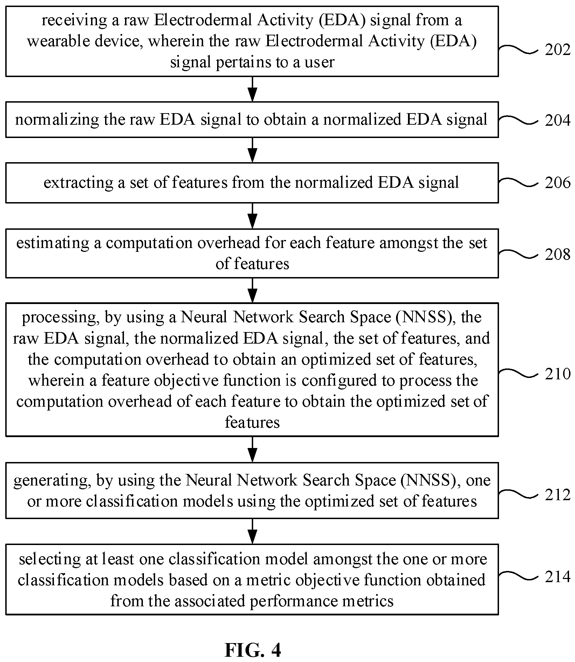

FIG. 4, with reference to FIGS. 1-3, depicts an exemplary flow chart illustrating a method for feature optimization and automated generation of tiny models for affective parameter determination from electrodermal activity, using the systems 100 of FIGS. 1-2, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, the block diagram of the system 100 depicted in FIGS. 2 and 3, and the flow diagram as depicted in FIG. 4. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

At step 202 of the method of the present disclosure, the one or more hardware processors 104 receive a raw Electrodermal Activity (EDA) signal from a wearable device. The raw Electrodermal Activity (EDA) signal captured pertains to a user associated with the wearable device. For instance, the raw EDA signal is captured when the user has worn the wearable device.

At step 204 of the method of the present disclosure, the one or more hardware processors 104 normalize the raw EDA signal to obtain a normalized EDA signal. To normalize the raw EDA signal, at first one or more baseline components in the raw EDA signal are determined. Then mean and standard deviation for the one or more baseline components are computed based on presence of an activity or stimuli in the raw EDA signal to obtain the normalized EDA signal. The steps 202 and 204 are better understood by way of following description.

A collection of datasets has been used, namely WESAD (e.g., refer "P. Schmidt, A. Reiss, R. Duerichen, C. Marberger, and K. Van Laerhoven, "Introducing wesad, a multimodal dataset for wearable stress and affect detection," in Proceedings of the 20th ACM international conference on multimodal interaction, 2018, pp. 400-408."), Affective Road (e.g., refer "N. E. Haouij, J.-M. Poggi, S. Sevestre-Ghalila, R. Ghozi, and M. Ja ̈ı dane, "Affectiveroad system and database to assess driver's attention," in Proceedings of the 33rd Annual ACM Symposium on Applied Computing, ser. SAC '18. New York, NY, USA: Association for Computing Machinery, 2018, p. 800-803. [Online]. Available: https://doi.org/10.1145/3167132.3167395"), S-Test (e.g., refer "M. Gjoreski and et al., "Monitoring stress with a wrist device using context," Journal of biomedical informatics, vol. 73, p. 159, 2017.") and DS-3 (e.g., refer "R. D. Gavas and et al., "A sensor-enabled digital trier social stress test in an enterprise context," in IEEE EMBC, 2019, p. 1321."). Each dataset has been divided into two parts train and test subjects following the x:y split ratio (e.g., in this case 90:10 split ratio). This amounts to a total of Stress and No-Stress data from 60 subjects, with 52 and 8 subjects in train and test sets respectively. One or more signal processing methods as known in the art were implemented by the system 100 and the method of the present disclosure to validate the data, down-sample it (if necessary) to 'p' Hertz (e.g., say 4 Hertz), and performed subject-specific baseline normalization. Following this step, bandpass filtering was applied, and the data was windowed into 30-second instances. Subsequently, the process includes handcrafted feature generation, feature selection, and finally machine learning-based classification. All the datasets have some baseline data followed by a task stressor, to impart stress condition on the subject. To build a classification model, the data belonging to the baseline segment is used as No-Stress class. Similarly, data from the task segment is taken to be from the Stress class. Given the data rate as 4Hz and window size of 30 seconds, the distribution of the number of instances across No-Stress and Stress is given in Table 1. More specifically, Table 1 illustrates distribution of instances across stress labels. It is also to be noted, that for all evaluations, subject-specific baseline normalized data has been used. The system 100 and method of the present disclosure aim to automate the time-consuming process of signal processing and come up with a model that is better or comparable in performance, while ensuring it is a tiny model capable of running on resource-constrained edge devices.

Table 1

| Partition | Subjects | No-Stress | Stress | Total |
|-----------|----------|-----------|--------|-------|
| Train | 52 | 1134 | 1495 | 2629 |
| Test | 8 | 178 | 243 | 421 |

At step 206 of the method of the present disclosure, the one or more hardware processors 104 extract a set of features from the normalized EDA signal. The set of features comprises at least one of one or more Tonic features, one or more Phasic features, a ratio of a mean associated with the one or more Tonic features and the one or more Phasic features, an entropy ratio of the one or more Tonic and the one or more Phasic features, a Phasic features peak from an associated baseline and an associated threshold, a Tonic features peak from an associated baseline and an associated threshold, a fractal dimension of the one or more Phasic features, a skin conductance stimuli, an associated slope obtained from the normalized EDA signal, and one or more frequency domain features.

The input raw EDA signal is normalized and processed, and a set of custom features are computed to enable the selection of the correct subset of features, and to ensure that the resulting models are optimized in terms of both input size, as well as model architecture. In addition to this, feature subsets are also selected based on the computational complexity involved in computing each of the features. This is an important factor since the feature computation overhead is an essential component in the case of real-time deployment, and it factors into the overall latency of the system. The definitions of the custom features and the concept of feature computation complexity are provided in the subsections below.

Tonic Features: The tonic features refer to the component of the EDA signal denoting long-term fluctuations and may be characterized by changes in the skin conductance level. The tonic features relate to slow-acting components, including various background characteristics depicted in the signal. The tonic feature is estimated with A Low pass filter for 0.1Hz cutoff.

Phasic Features: The phasic features refer to the component of the EDA signal denoting certain sudden transient events as a result of either any underlying sympathetic reaction which is evoked due to the presence of a stimulus, often referred to as 'event-related skin conductance responses', or due to spontaneous responses in the EDA signal. This is estimated by $S_p = S - S_t$; $S$ =complete signal, and $S_j$ = $j$th data point.

Ratio of a mean associated with the one or more Tonic features and the one or more Phasic features: This is defined as the ratio of the mean value of tonic component of the EDA signal to the mean value of phasic component of the EDA signal and is given by the expression $[(\frac{(1/n)\sum s_t}{1/n \sum s_p})$.

Entropy ratio of the one or more Tonic and the one or more Phasic features: The ratio of entropy of the tonic and phasic components reflects the nature of stimulus (sudden or prolonged) or activity to which the subject is exposed. This value varies with the different affective parameter targets. Entropy is computed by the expression $H(S_x) = - \sum_w p(S_{xj}) \log_2 (p(s_{xj}))$; Tonic entropy $H_t = H(S_t)$; Phasic Entropy $H_p = H(S_p)$;

$$\text{Entropy Ratio of Tonic and Phasic features} = H_t / H_p .$$

Phasic peak from baseline ($S_{pp}$) and associated threshold ($S_{pp\_t}$): The value of this feature signifies magnitude of response to a sudden stimulus in a given window. The threshold value can be preset to a suitable value, e.g., $S_{pp\_t}$ = 0.6, which means that the feature is considered impactful if the value is at least 60% of the maximum amplitude. Phasic component signal $S_p$, window length $t_w$, $S_{pp} = \max(S_p[t, t + t_w])$-min $(S_p[t, t + t_w])$.

Tonic peak from baseline ($S_{tp}$) and associated threshold ($S_{tp\_t}$): The value of this feature signifies magnitude of response to a prolonged stimulus in a given window. The threshold value can be preset to a suitable value, e.g., $S_{tp\_t}$ = 0.8, which means that the feature is considered impactful if the value is at least 80% of the maximum amplitude. Tonic component signal $S_t$, window length $t_w$, $S_{tp} = max(S_t[t, t + t_w])$ - mean $(S_t[t, t + t_w])$.

Fractal dimension: Fractal analysis to quantify the complexity or irregularity of the skin conductance, which are associated with stress responses. The system 100 calculates Nphasic ($\varepsilon$) for various box sizes, creating a log-log plot, and then estimates the slope of the linear regression line fitted to the dataset to obtain $D_{phasic}$. Fractal dimension of the phasic component $D_{phasic} = lim \, \varepsilon \to 0 \log(Nphasic(\varepsilon))/\log(1/\varepsilon)$. $\varepsilon$ is the size of the boxes used to cover the phasic component; $Nphasic(\varepsilon)$ is the number of boxes of size $\varepsilon$ needed to cover the phasic component.

Skin conductance:
$$ZCR = \left(\frac{1}{w-1}\right) * \sum(1 \ if \ S_j * S_{j-1} < 0, else \ 0)$$
, Slope = $(S_j - S_{j-1})/\Delta t$.

Accumulated GSR: This is denoted by the sum of the signal components of a given window, $\Sigma S_j [t, t + t_w]$. It is to be understood by a person having ordinary skill in the art or person skilled in the art that the system 100 is implemented and designed in such a way that it functions in case of larger superset of custom features as well.

Once the above set of features are extracted, at step 208 of the method of the present disclosure, the one or more hardware processors 104 estimate a computation overhead for each feature (e.g., also referred to as feature complexity and interchangeably used herein) amongst the set of features. The feature complexity is determined by the number of operations performed while computing a specific feature. For example, in case of raw EDA signal and subject-normalized EDA signal, $F_{ci} = 0$, since no feature is computed in these inputs. Another example can be considered in case of the ratio of tonic and phasic components, where the number of additions performed is equivalent to twice the window size-1, or 2*(n-1), and the number of multiplications performed is equal to 1. In this case, since only the multiplications and additions are considered as one of the important metrics in the design of tiny models, the feature complexity (computation overhead of the feature) can be expressed as $F_{ci} = f(N + mul_i + N + add\_i)$.

Referring to steps of FIG. 4, at step 210 of the method of the present disclosure, the one or more hardware processors 104 process, by using a Neural Network Search Space (NNSS), the raw EDA signal, the normalized EDA signal, the set of features, and the computation overhead to obtain an optimized set of features. A feature objective function is configured to process the computation overhead of each feature to obtain the optimized set of features. The NNSS is also referred to as a Neural Architecture Search and interchangeably used herein.

At step 212 of the method of the present disclosure, the one or more hardware processors 104 generate, by using the Neural Network Search Space (NNSS), one or more classification models using the optimized set of features. As an example, features used in the generation of classification models are illustrated below, wherein an input normalized EDA signal can be considered as follows:

S = [32.43814209, 48.53788612, 61.31122256, 68.82704475, 70.9144041 , 69.13274049, 65.84442959, 63.15539101, 62.1590975 , 62.75726417, 64.09917497, 65.23391374, 65.59113004, 65.19348115, 64.39243554, 63.29423225, 63.36185526, 63.22079352, 63.00968617, 62.76723238, 62.51454422, 62.28238963, 62.073584 , 61.88487031, 61.71124958, 61.52789044, 61.36503379, 61.25793606, 61.25672637, 61.40828902, 61.7405151 , 62.22956685, 62.81808346, 63.40337361, 63.88353559, 64.19187492, 64.28430858, 64.1926398 , 63.95766415, 63.68734808, 63.42765945, 63.24094306, 63.1364089 , 63.10825013, 63.13171859, 63.16743633, 63.19670485, 63.18831477, 63.11955289, 62.96498673, 62.73879533, 62.4737227 , 62.2598075 , 62.20015772, 62.38118036, 62.86140654, 63.60864495, 64.50881577, 65.37554294, 66.03383604, 66.35513138, 66.33584994, 66.05984081, 65.73017816, 65.55878942, 65.71011856, 66.2428785 , 67.09118879, 68.09953867, 69.06263497, 69.8228375 , 70.24753327, 70.33880688, 70.1235854 , 69.69698321, 69.14693552, 68.56954899, 68.01563784, 67.51804481, 67.06330744, 66.63324165, 66.21174077, 65.78571135, 65.38021948, 65.04677914, 64.84960319, 64.8597809 , 65.10616706, 65.59669285, 66.26991482, 67.03020589, 67.76678863, 68.40431012, 68.88017862, 69.17217169, 69.28941159, 69.24478303, 69.06324846, 68.76207356, 68.36181588, 67.88770597, 67.40516795, 66.95510555, 66.61448641, 66.39684646, 66.1654933 , 67.27684223, 68.13927662, 68.58081412, 68.1971629 , 66.95607917,65.47191835, 64.76970817, 65.76530888, 68.52741882, 71.91557731, 73.69149012, 71.42175737, 63.49702005, 50.13522548].

Window duration = 30 seconds, Sampling Rate = 4 Hz Window Size = 120 samples Tonic Features = LPF(S, Cut-off = 0.1 Hz); $S_t$ = LPF(S, 0.1).

=> $S_t$ = [32.65399457, 36.14706381, 39.57110947, 42.86165992, 45.96608293, 48.84614175, 51.47782562, 53.84918003, 55.95729394, 57.80552324, 59.40157082, 60.75648088, 61.88420612, 62.8013014 , 63.52644255, 64.07969948, 64.48168723, 64.75283411, 64.91291519, 64.98077392, 64.97410351, 64.90925857, 64.80110707, 64.66292476, 64.50632854, 64.34123959, 64.17586515, 64.01669215, 63.86849796, 63.73439557, 63.61593667, 63.5132938 , 63.4255303 , 63.35094578, 63.28746197, 63.23299847, 63.18578612, 63.14457876, 63.10874652, 63.0782598 , 63.05359521, 63.03560533, 63.02539068, 63.02420004, 63.03337031, 63.05430208, 63.08845723, 63.13736061, 63.20258771, 63.28572168, 63.38826438, 63.51149358, 63.65627231, 63.82283617, 64.01060361, 64.21806335, 64.44278623, 64.68158387, 64.9307957 , 65.18664211, 65.44555222, 65.70437565, 65.96041914, 66.2113037 , 66.45469683, 66.68801974, 66.90824181, 67.11184848, 67.29501525, 67.45395845, 67.58538361, 67.68693083, 67.75752495, 67.7975707 , 67.80897517, 67.79501684, 67.76010328, 67.70946821, 67.64885354, 67.5842077 , 67.521413 , 67.46603913, 67.42311105, 67.39688007, 67.39059462, 67.40628177, 67.44456522, 67.50455444, 67.58383733, 67.67859598, 67.78384271, 67.89374812, 68.00201286, 68.10223023, 68.18819487, 68.2541281 , 68.29480788, 68.30560616, 68.28244488, 68.22168487, 68.11996264, 67.97399221, 67.78035505, 67.53530948, 67.23465418, 66.87367168, 66.44720182, 65.94996885, 65.37720603, 64.72537558, 63.99269142, 63.17928497, 62.28707258, 61.31963785, 60.2825999 , 59.18482385, 58.04040428, 56.87076217, 55.70572313, 54.58237756]

$$\text{Phasic Features, } S_p = S - S_t$$

$S_p$ = [-2.15852481e-01, 1.23908223e+01, 2.17401131e+01, 2.59653848e+01, 2.49483212e+01, 2.02865987e+01, 1.43666040e+01, 9.30621098e+00, 6.20180356e+00, 4.95174094e+00, 4.69760415e+00, 4.47743285e+00, 3.70692392e+00, 2.39217975e+00, 8.65992984e-01, -7.85467226e-01, - 1.11983197e+00, -1.53204058e+00, -1.90322902e+00, -2.21354154e+00, - 2.45955929e+00, -2.62686895e+00, -2.72752306e+00, -2.77805446e+00, - 2.79507896e+00, -2.81334915e+00, -2.81083136e+00, -2.75875608e+00, - 2.61177158e+00, -2.32610656e+00, -1.87542158e+00, -1.28372694e+00, - 6.07446839e-01, 5.24278287e-02, 5.96073624e-01, 9.58876452e-01, 1.09852246e+00, 1.04806104e+00, 8.48917632e-01, 6.09088279e-01, 3.74064244e-01, 2.05337729e-01, 1.11018226e-01, 8.40500909e-02, 9.83482857e-02, 1.13134251e-01, 1.08247616e-01, 5.09541579e-02, -8.30348194e-02, -3.20734954e-01, -6.49469058e-01, -1.03777088e+00, -1.39646481e+00, -1.62267844e+00, - 1.62942324e+00, -1.35665681e+00, -8.34141276e-01, -1.72768096e-01, 4.44747242e-01, 8.47193937e-01, 9.09579158e-01, 6.31474292e-01, 9.94216759e-02, -4.81125535e-01, -8.95907403e-01, -9.77901181e-01, -6.65363317e-01, - 2.06596838e-02, 8.04523422e-01, 1.60867652e+00, 2.23745389e+00, 2.56060244e+00, 2.58128193e+00, 2.32601470e+00, 1.88800804e+00, 1.35191868e+00, 8.09445710e-01, 3.06169628e-01, -1.30808731e-01, -5.20900255e-01, -8.88171353e-01, -1.25429836e+00, -1.63739970e+00, -2.01666059e+00, - 2.34381549e+00, -2.55667858e+00, -2.58478432e+00, -2.39838738e+00, - 1.98714449e+00, -1.40868116e+00, -7.53636819e-01, -1.26959496e-01, 4.02297255e-01, 7.77948392e-01, 9.83976817e-01, 1.03528348e+00, 9.49975149e-01, 7.57642295e-01, 4.79628674e-01, 1.40131011e-01, -2.32256672e-01, - 5.68824259e-01, -8.25249494e-01, -9.20823071e-01, -8.37807717e-01, - 7.08178384e-01, 8.29640407e-01, 2.18930777e+00, 3.20360809e+00, 3.47178732e+00, 2.96338776e+00, 2.29263338e+00, 2.48263558e+00, 4.44567103e+00, 8.24481891e+00, 1.27307535e+01, 1.56510858e+01, 1.45509952e+01, 7.79129692e+00, -4.44715209e+00].
Ratio of Tonic component mean to Phasic component mean = 40.277741243537044 Phasic Peak from baseline, $S_{pp}$ = 30.412536922175775 Scaled $S_{pp}$ = 0.04574236370500584 Tonic Peak from baseline, $S_{pt}$ = 4.902166287956632 Entropy Ratio = 4.782136137758949. A subset of the above-calculated features are input to the classification model at the model optimization step (e.g., fine-tuning of the generated classification models) as depicted in the FIG. 3.

At step 214 of the method of the present disclosure, the one or more hardware processors 104 select at least one classification model amongst the one or more classification models based on a metric objective function obtained from the associated performance metrics. The above steps 210 through 214 are better understood by way of following description.

The system 100 aims to design customized classification networks (or also referred to as classification models/tiny models and interchangeably used herein) for on-device stress (affective parameter) detection from wearable EDA signals. To achieve this, the system 100 has employed a Reinforcement Learning (RL)-based NAS technique/algorithm, with the system 100 scaled down for expedited performance targeting smaller devices. Here, a maximum of 1000 episodes were used for optimization. The target size was kept at 200 kB but can be tuned according to requirements. Since the objective function plays a pivotal role in the search algorithm, the system 100 has modified the reward to add priority to the Accuracy metric, because the EDA signal has a very low frequency (e.g., 4 Hz) and the resulting model size of the sampled models are less than 1 MB. The metric objective function follows the expression:

$$O(A, S) = \frac{w_A \times (A - A_t) + w_S \times f(S - t_r * S_t)}{(w_A + w_S)} \qquad (1)$$

Where, $w_A$ and $w_S$ are priority weights for Accuracy and Model Size set to 2 and 1 respectively, $A_t$ and $S_t$ are target values to determine the exploration range of sampled models, $t_r$ denotes a target ratio for the Model Size metric, and set to 0.8 and $f$ is a non-linear function portraying the behavior of the objective function in the ranges $S < t_r \times S_t, t_r \times S_t <= S <= S_t, S > target_S$. The metric denoting the number of Multiply-Accumulate operations (MACs) was controlled by an upper threshold value $M_t$ depending on the computational capacity and battery power of the wearable device with the end-user. The MACs metric was optimized automatically with the Model Size-based objective.

Several other metrics need to be considered while optimizing a model for continuously running applications on edge devices. Some of these additional metrics are latency and power consumption, which can be substituted by surrogate metric MACs in a static scenario. For a more dynamic estimation, the device hardware specifications need to be considered.

Another important metric is the Peak Memory Usage (PMU), which is the highest memory requirement of a model, and is proportional to the highest value of layer-wise MACs estimated. All additional metrics can be incorporated into the objective function to achieve a multi-objective optimization and find the best model. In the implementation of the system and method of the present disclosure, the model size metric was sufficient to help in the quick convergence of the search algorithms. The above steps are further better understood by way of the following description:

The NAS algorithm is started with the initialization of affective parameter from the choices of say, stress, anxiety, emotions, cognitive load, attention, valence}, and the input of a labeled dataset with binary, or graded labels denoting the presence of level of affective parameter (e.g., Stress) for each signal input in the dataset. Next, a search space is defined, which can be either a large set of Neural Network Layers and parameters defining those layers, or it can be a smaller set refined through domain knowledge. This is a one-time step performed in the system 100 and is maintained constant throughout the run of the system, irrespective of the selected affective parameters. Next, the initialization of target device is done, which defines the device constraints utilized in the optimization algorithm. Next, the custom features (e.g., the set of features) are computed from the EDA signal, and simultaneously, the feature complexity is evaluated. All these components are direct or indirectly derived inputs which go into the NAS/NNSS.

In the first stage of optimization, feature combinations are sampled from the Feature superset, $F => F_s$, subset of F, followed by the sampling of models from the NN Search Space for each of the sampled Feature subsets $F_s$, in a randomized manner. Next, the sampled models are evaluated by partial or complete training with the accuracy, or an estimation of accuracy through matrix evaluation techniques. The metric objective function, $OM = f(accuracy, model\_size)$ is calculated, which is a function of the accuracy and model size (Multiply-accumulate operations, power, or latency can also be used in this case, as required by the application). Typically, the function constitutes a linear term of the accuracy or accuracy estimate, and a non-linear, exponential, or sigmoid function of the model footprint metric (e.g., model size) which has slower variance in values below a certain percentage of available memory space. For example, if the memory limit is 256 kB, the percentage can be set to 0.8, which is ~200 kB. So, the metric objective function degrades slowly from a maximum value (e.g., 1) when it has a value below 200 kB, then falls quickly in the range of 201 kB-256 kB and reaches 0 as soon as it crosses 256 kB. The linear function of accuracy or accuracy estimate adds a baseline component to the metric objective function. Next, the feature objective function is estimated as $OF = \Sigma Fcs$, where $Fcs$ is the sum of feature complexity corresponding to the feature subset $F_s$. The optimized feature set $F'$ (also referred to as optimized set of features) is estimated, where: $F' = F_s[argmin(OF)]$ from the sampled/classification models with top 10 metric objective values.

Following the optimization of features, the optimized feature subset $F'$ is utilized to further sample models from the NN Search Space. Here, the number of sampled models is larger than the number of sampled models in the feature optimization stage. The models are then evaluated and the metric objective $OM = f(accuracy, model\_size)$ is estimated again. It is to be understood by a person having ordinary skill in the art or person skilled in the art that other parameters such as other parameters mac_operations, latency, power, etc. can also be considered/implemented by the system 100 and the method of the present disclosure for feature optimization and models generation as per the requirement and such examples of performance metrics shall not be construed as limiting the scope of present disclosure. In the present disclosure, during generating the given results only two metrics have been considered in Metric Objective, and the MAC value, wherein MAC value was set to an upper threshold (=15 million operations), which is standard for the selected hardware target platform. It is to be understood by a person having ordinary skill in the art or person skilled in the art that such settings shall not be construed as limiting the scope of present disclosure. The value of metric objective function $OM$ is maximized by sampling models iteratively and evaluating the objective function. In the NAS/NNSS implementation, a Reinforcement Learning based optimization is utilized to achieve this. The final output of the system 100 is the fine-tuned model architecture with optimal features (also referred to as a selection of at least one classification model/tiny model amongst the one or more classification/tiny models). Once the classification model is selected and deployed in wearable device, the system 100 enables the selected classification model to determine, in real-time at the wearable device, a presence of a graded level of an affective parameter in the user based on an EDA signal being captured. As mentioned above, the affective parameter is at least one of stress, anxiety, emotion, cognitive load, attention, and valence.

After the fine-tuned model is obtained as output, the model can be further optimized through standard tools (e.g., TFLite converter as known in the art) and converted to a device-compatible version. In one implementation by the system 100, the selected target device is the Arduino Nano 33 BLE Sense TinyML board, which has an on-board RAM of 256 kB and a flash memory of 1 MB. It runs on 3.3 V input, and thus is a good simulation of extremely low power systems. On this device, the perceived affective parameter presence, or its level, as applicable, is denoted in the form of LED colors or text displays. In the stress detection system, 'No_Stress' can be denoted by a Green LED, and 'Stress' can be denoted by a blinking Red LED. The device can vary as per availability with the user, and so does the detection and indication and display of the affective parameter.

RESULTS and COMPARISON:

The system 100 and the method present comprehensive results obtained from experimentation of the present disclosure on the designated dataset. The distinct training and testing sets enable robust evaluation of various approaches, including traditional machine learning methods, deep learning models, and approaches stemming from NAS/NNSS. The subsequent analysis delves into the performance metrics such as accuracy, sensitivity, specificity, and f1-score. Given the aim of developing a model that can cater to the resource-constrained nature of edge devices, the system 100 further compared the models in terms of the model size and number of parameters and optimize the Multiply-Accumulate operations with the help of a threshold. This multifaceted evaluation provides a nuanced understanding of the effectiveness and generalization capabilities of the considered methodologies across diverse learning paradigms.

Performance of the State of the Art (SoA) Benchmark Model

After all the signal processing stages, the data is segmented into instances of 30-second windows. A list of 48 features is first engineered, and then a set of selected 40 features is then used to build a final classification model. The present disclosure further employed a combination of 3 classifiers namely Gradient Boosted trees, Random forest, and AdaBoost. The three classifiers were then combined using a voting mechanism based on the grid search of different combinatorial weights across 3 dimensions for the 3 classifiers. The best model is selected based on the best performance across all the different weight combinations. The final model (referred to as Base ML Model) is then used for performance evaluation.

Deep learning Models

Three deep learning models were used to evaluate the same train and test sets as discussed below. The first model is an open-sourced ResNet Model as given in literature (e.g., refer "Z. Wang, W. Yan, and T. Oates, "Time series classification from scratch with deep neural networks: A strong baseline," in 2017 International joint conference on neural networks (IJCNN). IEEE, 2017, pp. 1578-1585."), which is validated as a best-performing model over a collection of 85 UCR univariate time series data (e.g., refer "H. A. Dau, A. Bagnall, K. Kamgar, C.-C. M. Yeh, Y. Zhu, S. Gharghabi, C. A. Ratanamahatana, and E. Keogh, "The ucr time series archive," 2019."). This is a highly deep model with about 11 layers of Convolutional 1-dimensional units with intermediate gap layers (henceforth referenced as ResNet Conv1D). It is to be noted that the model has been used on an as-is basis with no hyperparameter tuning performed. Second, the system 100 used a basic 2-layered dense artificial neural network architecture (DenseANN 2L), comprising 64 and 32 neurons respectively. The final layer is the classification with 2 output neurons with softmax activation function and adam optimizer. Lastly, given the capability of LSTM models to work well with time series data, a 2-layered LSTM model with 120 and 60 units respectively (henceforth referred to as LSTM 2L) is used, followed by a dense ANN layer with 10 neurons and 2 neurons for the classification layer. Except for the last layer RELU activation function is used, followed by softmax activation in the last layer. To compute the loss categorical cross entropy is employed. All the deep learning models were trained for 100 epochs with a batch size of 16.

As described above, the performance of the base model i.e., weighted voting ensemble of classifiers Random Forest, Gradient boosted trees, and AdaBoost combined is reported in below Table 2 on an as-is basis from the ML benchmark approach (e.g., Performance Metrics of Different Models).

Table 2

| Model design | Mod el size | Paramet ers | Accura cy (%) | Sensitiv ity | Specifi city | Precisi on | Rec all | f1-sco re |
|---|---|---|---|---|---|---|---|---|
| Base_ML_m odel | 1.3 MB | | 89.1 | 0.87 | 0.92 | 0.93 | 0.87 | 0.9 0 |
| ResNet_Con v1D | 6.3 MB | 506818 | 71.50 | 0.51 | 0.99 | 0.99 | 0.51 | 0.6 7 |
| Dense_ANN _2L | 938. 6 kB | 9890 | 83.13 | 0.79 | 0.87 | 0.89 | 0.79 | 0.8 5 |
| LSTM_2L | 1.3 MB | 102632 | 84.80 | 0.80 | 0.91 | 0.92 | 0.80 | 0.8 6 |
| TinyStress 1 | 68.1 7 kB | 8034 | 85.75 | 0.82 | 0.91 | 0.92 | 0.82 | 0.8 7 |
| TinyStress 2 | 49.4 0 kB | 3682 | 85.98 | 0.82 | 0.92 | 0.93 | 0.82 | 0.8 7 |

Across all the different approaches reported, the base model reports the highest performance with an accuracy of 89.1% and an f1-score of 0.9. But has a considerable model size of 1.3 MB.

Performance of the Deep Learning Model as implemented by the system 100 and the method of the present disclosure. Although the system 100 has used 2 basic deep learning architectures (DenseANN 2L and LSTM 2L) and an open source moderately deep model Resnet Conv1D on an as-is basis without applying any hyper-parameter tuning, the performance of the model are close to Base ML Model. Further, except for DenseANN 2L, the number of training parameters is also quite high leading to a larger model size in the order of megabytes (MB). It is to be noted, that the system 100 did not perform any hyper-parameter tuning to boost the performance of the model of the present disclosure, as it may give better performance but eventually, the number of training parameters would remain the same leading to the same model size. Also, though deep learning models help the system 100 to skip the steps for traditional machine learning hence saving the time and effort of feature engineering and selection but have a high training time. On a CPU-based laptop the train time for DenseAnn 2L, LSTM 2L and Resnet Conv1D models were 26.4 seconds, 25.576 minutes, and 33.622 minutes respectively. Apart from this, a significant model design time is also required which is difficult to quantify.

Performance of the Auto-generated Models

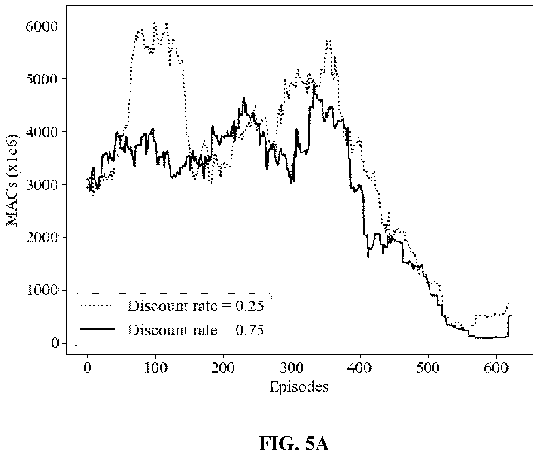

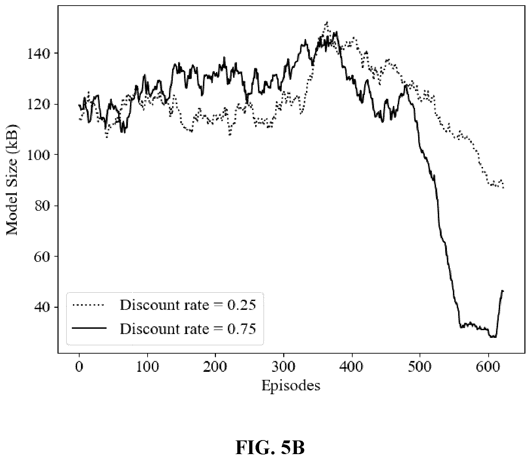

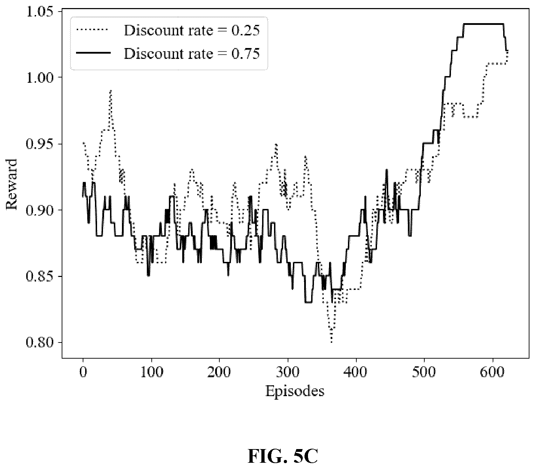

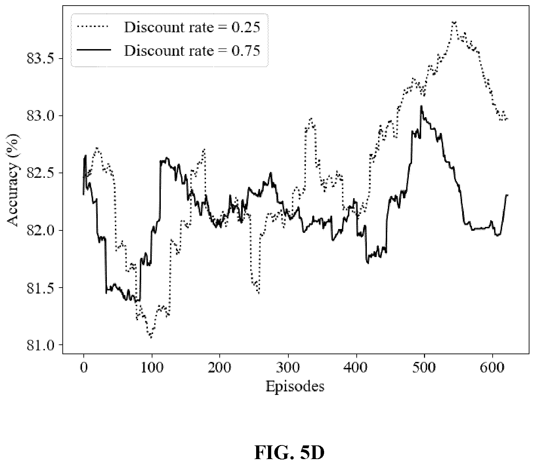

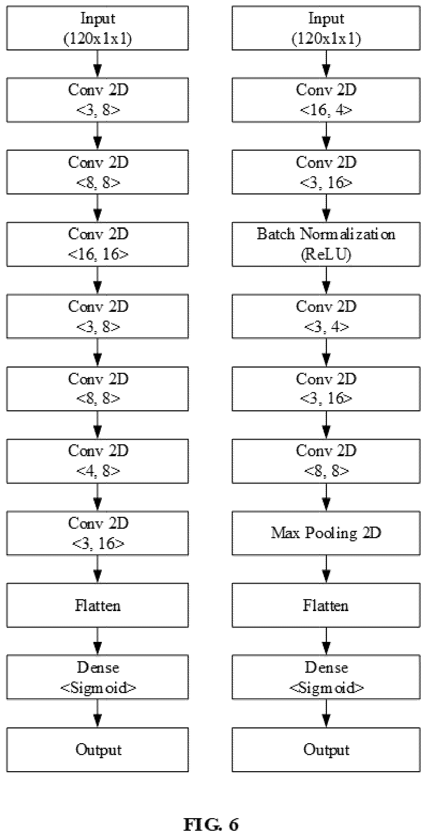

The model generation NAS framework was run for a maximum of 1000 episodes and required less than 2 hours to converge on a CPU-based laptop system. Instead of partial training of candidate models, an early-exit strategy was used with a maximum of 30 epochs per episode, and a stratified validation set of 10% of the training data was used to guide the training process. Final Model training and testing took approximately 10 minutes, owing to the tiny size of the models. The performance of the auto-generated models TinyStress 1 and TinyStress 2 of the system 100 of the present disclosure (depicted in FIG. 6) are presented in the above Table 2. FIG. 6, with reference to FIGS. 1 through 5D, depicts auto-generated tiny models (e.g., classification models) by the system 100 of FIGS. 1-2, in accordance with an embodiment of the present disclosure. It can be noted from the FIG. 6, that the models have unique architectures in terms of layers, as well as kernel structures, owing to the liberty provided by the automation technique. This helps the domain experts in augmenting their expertise with expanded possibilities. The model TinyStress 1 has a size of 68.17 kB, a total of 8034 parameters with an accuracy of 85.74% and the model TinyStress 2 has a size of 49.40 kB, with total model parameters of 3682 and an accuracy of 85.98%. The models perform a total of 15.726 M and 4.023 M Multiply-accumulate operations respectively, making them suitable for highly constrained applications with the requirement of continuous, real-time, on-device inference, resulting in round-the-clock stress monitoring without excessive draining of battery power. FIGS. 5A through 5D depicts the optimization of candidate models via the moving average of the reward objective and other performance metrics with the progress of NAS/NNSS episodes. More specifically, FIGS. 5A through 5D, with reference to FIGS. 1 through 4, depict a graphical representation illustrating performance metrics for stress detection model generation (e.g., classification model) with a Neural Network Search Space (NNSS) (or Neural Architecture Search - NAS) in accordance with an embodiment of the present disclosure.

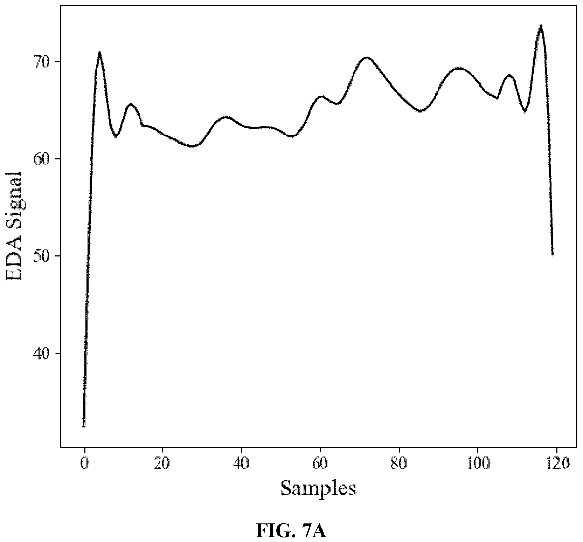

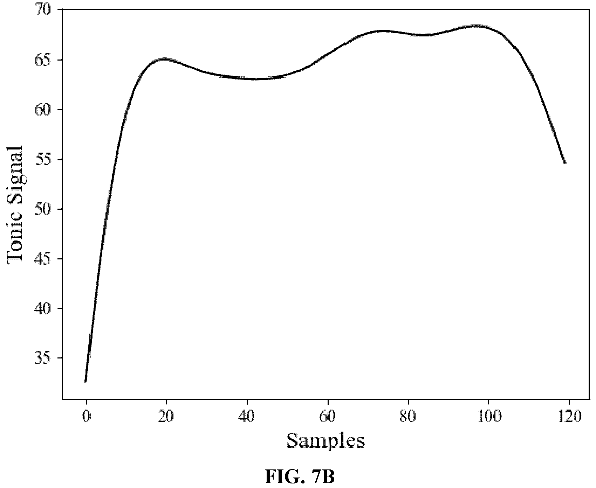

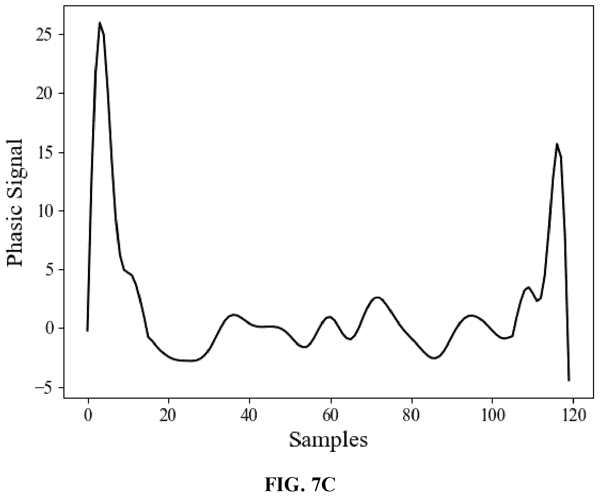

FIGS. 7A through 7C, with reference to FIGS. 1 through 6, depict a graphical representation illustrating selected time series features from a feature space (as opposed to features with one single value for a window), in accordance with an embodiment of the present disclosure. These signals are the input normalized signal $S$, Tonic features $S_t$, and Phasic features $S_p$ respectively. The optimized feature subset is divided into two parts - Time series and single valued features. The time series features are taken together with the normalized signal (in this example), and added at the input layer, changing the input dimension from <120 x 1 x 1> to <120 x 3 x 1>. The single values feature subset is taken, and after the Flatten () layer of the neural network, it is concatenated with the flattened features right before the final classification layer (Dense - Sigmoid layer) in this example. This is only an evaluated example. Based on random generations of the sampled models there is a scope to keep this position of feature concatenation as flexible (and not always before the classification layer), although not done in this example.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1.   A processor implemented method, comprising:

   receiving, via one or more hardware processors, a raw Electrodermal Activity (EDA) signal from a wearable device (202), wherein the raw Electrodermal Activity (EDA) signal pertains to a user;
   normalizing, via the one or more hardware processors, the raw EDA signal to obtain a normalized EDA signal (204);
   extracting, via the one or more hardware processors, a set of features from the normalized EDA signal (206);
   estimating, via the one or more hardware processors, a computation overhead for each feature amongst the set of features (208);
   processing, by using a Neural Network Search Space (NNSS) via the one or more hardware processors, the raw EDA signal, the normalized EDA signal, the set of features, and the computation overhead to obtain an optimized set of features, wherein a feature objective function is configured to process the computation overhead of each feature to obtain the optimized set of features (210);
   generating, by using the Neural Network Search Space (NNSS) via the one or more hardware processors, one or more classification models using the optimized set of features (212); and
   selecting, via the one or more hardware processors, at least one classification model amongst the one or more classification models based on a metric objective function obtained from the associated performance metrics (214).

2.   The processor implemented method as claimed in claim 1, wherein the step of normalizing the raw EDA signal comprises:

   determining one or more baseline components in the raw EDA signal; and
   computing mean and standard deviation for the one or more baseline components based on presence of an activity or stimuli in the raw EDA signal to obtain the normalized EDA signal.

3.   The processor implemented method as claimed in claim 1, further comprising determining in real-time at the wearable device, by using the at least one selected classification model deployed therein, a presence of a graded level of an affective parameter in the user based on an EDA signal being captured.

4. The processor implemented method as claimed in claim 3, wherein the affective parameter comprises at least one of stress, anxiety, emotion, cognitive load, attention, and valence.

5. The processor implemented method as claimed in claim 1, wherein the set of features comprises at least one of one or more Tonic features, one or more Phasic features, a ratio of a mean associated with the one or more Tonic features and the one or more Phasic features, an entropy ratio of the one or more Tonic and the one or more Phasic features, a Phasic features peak from an associated baseline and an associated threshold, a Tonic features peak from an associated baseline and an associated threshold, a fractal dimension of the one or more Phasic features, a skin conductance stimuli, an associated slope obtained from the normalized EDA signal, and one or more frequency domain features.

6. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive a raw Electrodermal Activity (EDA) signal from a wearable device, wherein the raw Electrodermal Activity (EDA) signal pertains to a user;
normalize, the raw EDA signal to obtain a normalized EDA signal;
extract a set of features from the normalized EDA signal;
estimate a computation overhead for each feature amongst the set of features;
process, by using a Neural Network Search Space (NNSS), the raw EDA signal, the normalized EDA signal, the set of features, and the computation overhead to obtain an optimized set of features, wherein a feature objective function is configured to process the computation overhead of each feature to obtain the optimized set of features;
generate, by using the Neural Network Search Space (NNSS) via the one or more hardware processors, one or more classification models using the optimized set of features; and
select at least one classification model amongst the one or more classification models based on a metric objective function obtained from the associated performance metrics.

7. The system as claimed in claim 6, wherein the raw EDA signal is normalized by:

determining one or more baseline components in the raw EDA signal; and
computing mean and standard deviation for the one or more baseline components based on presence of an activity or stimuli in the raw EDA signal to obtain the normalized EDA signal.

8. The system as claimed in claim 6, wherein the one or more hardware processors are further configured by the instructions to determine in real-time at the wearable device, by using the at least one selected classification model deployed therein, a presence of a graded level of an affective parameter in the user based on an EDA signal being captured.

9. The system as claimed in claim 8, wherein the affective parameter comprises at least one of stress, anxiety, emotion, cognitive load, attention, and valence.

10. The system as claimed in claim 6, wherein the set of features comprises at least one of one or more Tonic features, one or more Phasic features, a ratio of a mean associated with the one or more Tonic features and the one or more Phasic features, an entropy ratio of the one or more Tonic and the one or more Phasic features, a Phasic features peak from an associated baseline and an associated threshold, a Tonic features peak from an associated baseline and an associated threshold, a fractal dimension of the one or more Phasic features, a skin conductance stimuli, an associated slope obtained from the normalized EDA signal, and one or more frequency domain features.

11. One or more non-transitory machine-readable information storage mediums comprising one or more instructions which when executed by one or more hardware processors cause:

receiving a raw Electrodermal Activity (EDA) signal from a wearable device, wherein the raw Electrodermal Activity (EDA) signal pertains to a user;

normalizing the raw EDA signal to obtain a normalized EDA signal;

extracting a set of features from the normalized EDA signal;

estimating a computation overhead for each feature amongst the set of features;

processing, by using a Neural Network Search Space (NNSS), the raw EDA signal, the normalized EDA signal, the set of features, and the computation overhead to obtain an optimized set of features, wherein a feature objective function is configured to process the computation overhead of each feature to obtain the optimized set of features;

generating, by using the Neural Network Search Space (NNSS), one or more classification models using the optimized set of features; and

selecting at least one classification model amongst the one or more classification models based on a metric objective function obtained from the associated performance metrics.

12. The one or more non-transitory machine-readable information storage mediums as claimed in claim 11, wherein the step of normalizing the raw EDA signal comprises:

determining one or more baseline components in the raw EDA signal; and

computing mean and standard deviation for the one or more baseline components based on presence of an activity or stimuli in the raw EDA signal to obtain the normalized EDA signal.

13. The one or more non-transitory machine-readable information storage mediums as claimed in claim 11, wherein the one or more instructions which when executed by the one or more hardware processors further cause determining in real-time at the wearable device, by using the at least one selected classification model deployed therein, a presence of a graded level of an affective parameter in the user based on an EDA signal being captured.

14. The one or more non-transitory machine-readable information storage mediums as claimed in claim 13, wherein the affective parameter comprises at least one of stress, anxiety, emotion, cognitive load, attention, and valence.

15. The one or more non-transitory machine-readable information storage mediums as claimed in claim 11, wherein the set of features comprises at least one of one or more Tonic features, one or more Phasic features, a ratio of a mean associated with the one or more Tonic features and the one or more Phasic features, an entropy ratio of the one or more Tonic and the one or more Phasic features, a Phasic features peak from an associated baseline and an associated threshold, a Tonic features peak from an associated baseline and an associated threshold, a fractal dimension of the one or more Phasic features, a skin conductance stimuli, an associated slope obtained from the normalized EDA signal, and one or more frequency domain features.

SYSTEM
<u>100</u>

MEMORY
<u>102</u>

DATABASE
<u>108</u>

HARDWARE PROCESSOR(S)
<u>104</u>

INTERFACE(S)
<u>106</u>

**FIG. 1**

Base Model Design (Domain Expert)

Raw Data Acquisition

Feature Extraction

Design and Test of Model

Optimization for Wearable

EDA

Wearable Module

Automated Design (NAS/NNSS)

Raw Data Acquisition

Search Space Design

Auto-optimization

Wearable-ready Model

✔ On-device Inference

✔ Real-time Analysis

**FIG. 2**

FIG. 3

receiving a raw Electrodermal Activity (EDA) signal from a wearable device, wherein the raw Electrodermal Activity (EDA) signal pertains to a user — 202

normalizing the raw EDA signal to obtain a normalized EDA signal — 204

extracting a set of features from the normalized EDA signal — 206

estimating a computation overhead for each feature amongst the set of features — 208

processing, by using a Neural Network Search Space (NNSS), the raw EDA signal, the normalized EDA signal, the set of features, and the computation overhead to obtain an optimized set of features, wherein a feature objective function is configured to process the computation overhead of each feature to obtain the optimized set of features — 210

generating, by using the Neural Network Search Space (NNSS), one or more classification models using the optimized set of features — 212

selecting at least one classification model amongst the one or more classification models based on a metric objective function obtained from the associated performance metrics — 214

**FIG. 4**

**FIG. 5A**

**FIG. 5B**

**FIG. 5C**

**FIG. 5D**

**FIG. 6**

**FIG. 7A**

**FIG. 7B**

**FIG. 7C**

Europäisches
Patentamt

European
Patent Office

Office européen
des brevets

# EUROPEAN SEARCH REPORT

**Application Number**

EP 25 16 1457

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GIBBS MICHAEL ET AL: "Combining Multiple Tiny Machine Learning Models for Multimodal Context-Aware Stress Recognition on Constrained Microcontrollers", IEEE MICRO, IEEE SERVICE CENTER, LOS ALAMITOS, CA, US, vol. 44, no. 3, 1 November 2023 (2023-11-01), pages 67-75, XP011973434, ISSN: 0272-1732, DOI: 10.1109/MM.2023.3329218 [retrieved on 2023-11-02] | 1,2,5-7, 10-12,15 | INV. G16H50/20 A61B5/0533 A61B5/16 ADD. G06N3/08 |
| A | * the whole document * | 3,4,8,9, 13,14 | |
| | ----- | | |
| X | RAGAV ABHIJITH ET AL: "Scalable Deep Learning for Stress and Affect Detection on Resource-Constrained Devices", 2019 18TH IEEE INTERNATIONAL CONFERENCE ON MACHINE LEARNING AND APPLICATIONS (ICMLA), IEEE, 16 December 2019 (2019-12-16), pages 1585-1592, XP033719810, DOI: 10.1109/ICMLA.2019.00261 [retrieved on 2020-02-13] | 1,2,5-7, 10-12,15 | |
| A | * the whole document * | 3,4,8,9, 13,14 | |
| | ----- | | |
| | -/-- | | |

**TECHNICAL FIELDS SEARCHED (IPC)**

G16H
A61B
G06N

The present search report has been drawn up for all claims

| Place of search | Date of completion of the search | Examiner |
|---|---|---|
| Munich | 7 July 2025 | Lorenz, Larissa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 614 516 A1**

Europäisches Patentamt
European Patent Office
Office européen des brevets

# EUROPEAN SEARCH REPORT

Application Number

EP 25 16 1457

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Pham Nhat: "Enabling human physiological sensing by leveraging intelligent head-worn wearable systems" In: "Doctoral Thesis, University of Oxford", 1 December 2022 (2022-12-01), XP093291517, Retrieved from the Internet: URL:https://ora.ox.ac.uk/objects/uuid:0225 8612-9d32-443d-ac79-8a4be1609d3e/files/d1j 92g809b> * page 5 - page 6 * * pages 17, 23 * * paragraph [0065] - paragraph [0094] * | 1-15 | |
| A | MUKHOPADHYAY SHALINI ET AL: "Generating Tiny Deep Neural Networks for ECG Classification on Micro-Controllers", 2023 IEEE INTERNATIONAL CONFERENCE ON PERVASIVE COMPUTING AND COMMUNICATIONS WORKSHOPS AND OTHER AFFILIATED EVENTS (PERCOM WORKSHOPS), IEEE, 13 March 2023 (2023-03-13), pages 392-397, XP034362212, DOI: 10.1109/PERCOMWORKSHOPS56833.2023.10150311 [retrieved on 2023-06-21] * the whole document * | 1-15 | **TECHNICAL FIELDS SEARCHED (IPC)** |
| A | US 2021/319894 A1 (SOBOL ADAM G [US] ET AL) 14 October 2021 (2021-10-14) * paragraph [0219] - paragraph [0279] * | 1-15 | |

The present search report has been drawn up for all claims

| Place of search | Date of completion of the search | Examiner |
|---|---|---|
| Munich | 7 July 2025 | Lorenz, Larissa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

30

## ANNEX TO THE EUROPEAN SEARCH REPORT
## ON EUROPEAN PATENT APPLICATION NO.

EP 25 16 1457

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2025

| Patent document cited in search report | Publication date | Patent family member(s) | Publication date |
|---|---|---|---|
| US 2021319894 A1 | 14-10-2021 | US 2021319894 A1<br>US 2024274285 A1 | 14-10-2021<br>15-08-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IN 202421015894

### Non-patent literature cited in the description

- **M. PANTIC** ; **L. J. ROTHKRANTZ**. Toward an affect-sensitive multimodal human-computer interaction. *Proceedings of the IEEE*, 2003, vol. 91 (9), 1370-1390
- **R. A. CALVO** ; **S. D'MELLO**. Frontiers of affect-aware learning technologies. *IEEE Intelligent Systems*, 2012, vol. 27 (6), 86-89
- **P. SCHMIDT** ; **A. REISS** ; **R. D··URICHEN** ; **K. VAN LAERHOVEN**. Wearable-based affect recognition-a review. *Sensors*, 2019, vol. 19 (19), 4079
- **R. LI** ; **Z. LIU**. Stress detection using deep neural networks. *BMC Medical Informatics and Decision Making*, 2020, vol. 20, 1-10
- Stress detection with machine learning and deep learning using multimodal physiological data. **P. BOBADE** ; **M. VANI**. 2020 Second International Conference on Inventive Research in Computing Applications (ICIRCA). IEEE, 2020, 51-57
- **C. SETZ** ; **B. ARNRICH** ; **J. SCHUMM** ; **R. LA MARCA** ; **G. TR··OSTER** ; **U. EHLERT**. Discriminating stress from cognitive load using a wearable eda device. *IEEE Transactions on information technology in biomedicine*, 2009, vol. 14 (2), 410-417
- **S. A. KHOWAJA** ; **A. G. PRABONO** ; **F. SETIAWAN** ; **B. N. YAHYA** ; **S.-L. LEE**. Toward soft real-time stress detection using wrist-worn devices for human workspaces. *Soft Computing*, 2021, vol. 25, 2793-2820
- Real-time mental stress detection based on smartwatch. **L. CIABATTONI** ; **F. FERRACUTI** ; **S. LONGHI** ; **L. PEPA** ; **L. ROMEO** ; **F. VERDINI**. 2017 IEEE International Conference on Consumer Electronics (ICCE). IEEE, 2017, 110-111
- **F. HUTTER** ; **L. KOTTHOFF** ; **J. VANSCHOREN**. Automated machine learning: methods, systems, challenges. *Springer Nature*, 2019
- **B. ZOPH** ; **Q. V. LE**. Neural architecture search with reinforcement learning. *arXiv:1611.01578*, 2016
- **B. BAKER** ; **O. GUPTA** ; **N. NAIK** ; **R. RASKAR**. *Designing neural network architectures using reinforcement learning*, 2016
- **E. REAL** ; **S. MOORE** ; **A. SELLE** ; **S. SAXENA** ; **Y. L. SUEMATSU** ; **J. TAN** ; **Q. V. LE** ; **A. KURAKIN**. Large-scale evolution of image classifiers. *international conference on machine learning. PMLR*, 2017, 2902-2911
- **C. LI** ; **J. PENG** ; **L. YUAN** ; **G. WANG** ; **X. LIANG** ; **L. LIN** ; **X. CHANG**. Block-wisely supervised neural architecture search with knowledge distillation. *Proceedings of the IEEE/CVF Conference on Computer Vision and Pattern Recognition*, 2020, 1989-1998
- **M. ZHANG** ; **H. LI** ; **S. PAN** ; **X. CHANG** ; **S. SU**. Overcoming multi-model forgetting in one-shot NAS with diversity maximization. *Proceedings of the IEEE/CVF Conference on Computer Vision and Pattern Recognition*, 2020, 7809-7818
- Rnts: robust neural temporal search for time series classification. **Z. XIAO** ; **X. XU** ; **H. XING** ; **R. QU** ; **F. SONG** ; **B. ZHAO**. 2021 International Joint Conference on Neural Networks (IJCNN). IEEE, 2021, 1-8
- Neural architecture search for time series classification. **H. RAKHSHANI** ; **H. I. FAWAZ** ; **L. IDOUM-GHAR** ; **G. FORESTIER** ; **J. LEPAGNOT** ; **J. WEBER** ; **M. BR'EVILLIERS** ; **P.-A. MULLER**. 2020 International Joint Conference on Neural Networks (IJCNN). IEEE, 2020, 1-8
- Autotransformer: Automatic transformer architecture design for time series classification. **Y. REN** ; **L. LI** ; **X. YANG** ; **J. ZHOU**. Pacific-Asia Conference on Knowledge Discovery and Data Mining. Springer, 2022, 143-155
- **S. MUKHOPADHYAY** ; **S. DEY** ; **P. GHOSE** ; **A. ANS SINGH** ; **P. DASGUPTA**. Generating tiny deep neural networks for ecg classification on microcontrollers. *Proceedings of the 2023 IEEE International Conference on Pervasive Computing and Communications Workshops and other Affiliated Events (PerCom Workshops): PerCom Industry Track 2023*, 2023

- Puffconv: A system for online and on-device puff detection for smoking cessation. **V. SHARMA** ; **S. MUKHOPADHYAY** ; **S. BHATTACHARYA** ; **S. DEY** ; **A. GHOSE**. 2023 IEEE International Conference on Pervasive Computing and Communications Workshops and other Affiliated Events (PerCom Workshops). IEEE, 2023, 595-600
- **P. SCHMIDT** ; **A. REISS** ; **R. DUERICHEN** ; **C. MARBERGER** ; **K. VAN LAERHOVEN**. Introducing wesad, a multimodal dataset for wearable stress and affect detection. *Proceedings of the 20th ACM international conference on multimodal interaction*, 2018, 400-408
- **M. GJORESKI et al.** Monitoring stress with a wrist device using context. *Journal of biomedical informatics*, 2017, vol. 73, 159
- **R. D. GAVAS et al.** A sensor-enabled digital trier social stress test in an enterprise context. *IEEE EMBC*, 2019, 1321
- Time series classification from scratch with deep neural networks: A strong baseline. **Z. WANG** ; **W. YAN** ; **T. OATES**. 2017 International joint conference on neural networks (IJCNN). IEEE, 2017, 1578-1585
- **H. A. DAU** ; **A. BAGNALL** ; **K. KAMGAR** ; **C.-C. M. YEH** ; **Y. ZHU** ; **S. GHARGHABI** ; **C. A. RATANA-MAHATANA** ; **E. KEOGH**. *The ucr time series archive*, 2019